# EUROPEAN PATENT SPECIFICATION

(11) **EP 1 910 527 B1**
(45) Date of publication and mention of the grant of the patent: **27.02.2013**
(21) Application number: 05770325.8
(22) Date of filing: 05.08.2005
(51) Int. Cl.: C12N 9/12, C12P 19/00, C12N 5/10

(54) **MALTOSE-1-PHOSPHATE-PRODUCING ENZYME**
MALTOSE-1-PHOSPHAT PRODUZIERENDES ENZYM
ENZYME PRODUISANT DU MALTOSE-1-PHOSPHATE

(43) Date of publication of application: 16.04.2008
(73) Proprietor: Kao Corporation, Chuo-ku Tokyo 103-8210 (JP)
(72) Inventor: IGARASHI, Kazuaki, Tochigi 321-3497 (JP); TAKIZAWA, Shuichi, Tochigi 321-3497 (JP); HIGAKI, Norihiko, Tochigi 321-3497 (JP); HITOMI, Jun, Tochigi 321-3497 (JP)
(74) Representative: HOFFMANN EITLE
(86) International application number: PCT/JP2005/014856
(87) International publication number: WO 2007/017954

(56) References cited:
- EP-A- 1 108 790
- WO-A-01/00844
- WO-A-2005/111206
- DATABASE UniProt [Online] 5 July 2004 (2004-07-05), "Putative ALPHA-AMYLASE (EC 3.2.1.1)." XP002371419 retrieved from EBI accession no. UNIPROT:Q6M5V8 Database accession no. Q6M5V8
- DATABASE UniProt [Online] 1 March 2003 (2003-03-01), "Hypothetical protein." XP002371420 retrieved from EBI accession no. UNIPROT:Q8FQ11 Database accession no. Q8FQ11
- NIEHUES B ET AL: "Isolation and characterization of maltokinase (ATP:maltose 1-phosphotransferase) from Actinoplanes missouriensis" ARCHIVES OF MICROBIOLOGY, BERLIN, DE, vol. 180, no. 4, October 2003 (2003-10), pages 233-239, XP002346641 ISSN: 0302-8933

### SEQUENCE LISTING

<110> KAO CORPORATION
<120> Maltose-1-phosphate-producing enzyme
<130> KS0865
<160> 10
<170> PatentIn version 3.1
<210> 1
   <211> 2556
   <212> DNA
   <213> Corymebacterium glutamicum JCM1321
<220>
   <221> CDS
   <222> (247).. (2271).
   <223>
<400> 1
   ggagagaatt cgtcattgag ttcactcgaa tcagagggaa aagatgcagc tgcagagtta     60
   tttaaagagt tagtcacatc tcccatcttg actgatttct ccggttccgt gtggaagtga    120
   cacagctccg ttaagctttc ataatggaga catacccccg gctaattgcc ggtgtgtctg    180
   cttgatcgaa cttttaagct tgtgtgcgtt tgttcgcact tcacactgca tgttgaggag    240
   aaaact gtg act ggc cgt ctc ggt atc gat gat gtt cgt ccc cgg att       288
          Val Thr Gly Arg Leu Gly Ile Asp Asp Val Arg Pro Arg Ile
          1               5                   10
   ttg gat gga aat cca gcg aaa gca gta gtg ggt gag att gtt cct gtc      336
   Leu Asp Gly Asn Pro Ala Lys Ala Val Val Gly Glu Ile Val Pro Val
   15                  20                  25                  30
   tcg gct att gtg tgg cgt gaa ggc cat gat gca att gcc gcg acg ttg      384
   Ser Ala Ile Val Trp Arg Glu Gly His Asp Ala Ile Ala Ala Thr Leu
                   35                  40                  45
   aat gtt tcc ggt ccg gag gat tct tca gtg gcg gct gag cct att cag      432
   Asn Val Ser Gly Pro Glu Asp Ser Ser Val Ala Ala Glu Pro Ile Gln
               50                  55                  60
   att cat atg cgc ccc act cct gat aat cag gat cag tcg aat gca ttt      480
   Ile His Met Arg Pro Thr Pro Asp Asn Gln Asp Gln Ser Asn Ala Phe
           65                  70                  75
   ttc gtt cct gat gtc cct ggt aat tgg aca ttt cgt gtg gat gcg tgg      528
   Phe Val Pro Asp Val Pro Gly Asn Trp Thr Phe Arg Val Asp Ala Trp
       80                  85                  90
   tcg gat cct atg gct aca tgg cgt cat gcg att acc act aaa att gag      576
   Ser Asp Pro Met Ala Thr Trp Arg His Ala Ile Thr Thr Lys Ile Glu
   95                  100                 105                 110
   gca ggc cag ggt tca gat gag ttg tat aac gac ttc gag cac ggg gct      624
   Ala Gly Gln Gly Ser Asp Glu Leu Tyr Asn Asp Phe Glu His Gly Ala
                   115                 120                 125
   cag ctg ttt gaa cgg gct gcg gag aat ttg tct aaa gag gat agg act      672
   Gln Leu Phe Glu Arg Ala Ala Glu Asn Leu Ser Lys Glu Asp Arg Thr
               130                 135                 140
   gcg ctt ttc gac gtc gcc tcc tct ctg cgg cgc ggc ggc gat gta cgc      720
   Ala Leu Phe Asp Val Ala Ser Ser Leu Arg Arg Gly Gly Asp Val Arg
           145                 150                 155
   gca cgt ctc gcc cca gcg ctc acc tcg agt gtc act cat ctt tta gaa      768
   Ala Arg Leu Ala Pro Ala Leu Thr Ser Ser Val Thr His Leu Leu Glu
       160                 165                 170
   ctt aac ccg ttg cgt gag ttg gtc acg atg ggt gaa aac ctg cag gtt      816
   Leu Asn Pro Leu Arg Glu Leu Val Thr Met Gly Glu Asn Leu Gln Val
   175                 180                 185                 190
   cgt gtc gag cgt cgt gcc gct ttg gtc aac tct tgg tat gag ctt ttc      864
   Arg Val Glu Arg Arg Ala Ala Leu Val Asn Ser Trp Tyr Glu Leu Phe
   195 200 205
   cct cgt tcc aca ggt ggt tgg gat gag tcc ggc acc ccc gtt cat ggc 912
   Pro Arg Ser Thr Gly Gly Trp Asp Glu Ser Gly Thr Pro Val His Gly
   210 215 220
   act ttc gct acc act gct cag gcg ttg gag cgt gtc gcg aag atg ggc 960
   Thr Phe Ala Thr Thr Ala Gln Ala Leu Glu Arg Val Ala Lys Met Gly
   225 230 235
   ttc gat act gtt tac ttc ccg ccg atc cat ccg att ggc gag gtc aac 1008
   Phe Asp Thr Val Tyr Phe Pro Pro Ile His Pro Ile Gly Glu Val Asn
   240 245 250
   cgc aag ggc cgc aat aat acg ctg acc ccg gaa cct cat gat gtg ggt 1056
   Arg Lys Gly Arg Asn Asn Thr Leu Thr Pro Glu Pro His Asp Val Gly
   255 260 265 270
   tcg ccg tgg gct att ggt tct aaa gat ggt ggg cat gat gca acg cat 1104
   Ser Pro Trp Ala Ile Gly Ser Lys Asp Gly Gly His Asp Ala Thr His
   275 280 285
   ccg cgg ttg ggc acc att gaa gat ttc cag gcg ttg ttg gct cgc gca 1152
   Pro Arg Leu Gly Thr Ile Glu Asp Phe Gln Ala Leu Leu Ala Arg Ala
   290 295 300
   cgg gaa ctc aat ttg gaa gtt gca ctc gat cta gct ctg cag gct gcc 1200
   Arg Glu Leu Asn Leu Glu Val Ala Leu Asp Leu Ala Leu Gln Ala Ala
   305 310 315
   cct gat cat cca tgg gcg cag gaa cac cgc gag ttt ttc acg gtg ttg 1248
   Pro Asp His Pro Trp Ala Gln Glu His Arg Glu Phe Phe Thr Val Leu
   320 325 330
   gct gat ggc acc att gcg tat gca gaa aac cca ccg aag aag tac cag 1296
   Ala Asp Gly Thr Ile Ala Tyr Ala Glu Asn Pro Pro Lys Lys Tyr Gln
   335 340 345 350
   gat att tat ccc atc aac ttt gat aat gct gct ccg agg atc tat gaa 1344
   Asp Ile Tyr Pro Ile Asn Phe Asp Asn Ala Ala Pro Arg Ile Tyr Glu
   355 360 365
   gag gtc tat cgt gtg gtg aag ttc tgg gtg gat ttg ggt gtg acc aca 1392
   Glu Val Tyr Arg Val Val Lys Phe Trp Val Asp Leu Gly Val Thr Thr
   370 375 380
   ttc cgc gtg gat aac ccg cac act aag ccc gct aat ttc tgg cag tgg 1440
   Phe Arg Val Asp Asn Pro His Thr Lys Pro Ala Asn Phe Trp Gln Trp
   385 390 395
   ctt att tct gcc atc cat aaa tca aac cct gag gtc att ttc cta gcg 1488
   Leu Ile Ser Ala Ile His Lys Ser Asn Pro Glu Val Ile Phe Leu Ala
   400 405 410
   gag gcg ttt act cgc ccg gca cgt ctg tat ggc ttg gcc aag att ggt 1536
   Glu Ala Phe Thr Arg Pro Ala Arg Leu Tyr Gly Leu Ala Lys Ile Gly
   415 420 425 430
   ttc tcc cag tct tac acc tac ttc acg tgg aag gtc acc aag gag gag 1584
   Phe Ser Gln Ser Tyr Thr Tyr Phe Thr Trp Lys Val Thr Lys Glu Glu
   435 440 445
   ctc acc gag ttc gct act gag atc gcc cgc atg gcg gat att tct cgt 1632
   Leu Thr Glu Phe Ala Thr Glu Ile Ala Arg Met Ala Asp Ile Ser Arg
   450 455 460
   ccg aac ctg ttt gtg aac act ccc gac att ttg cat gcg tct ctg cag 1680
   Pro Asn Leu Phe Val Asn Thr Pro Asp Ile Leu His Ala Ser Leu Gln
   465 470 475
   cat ggt gga cgc gca atg ttc gct atc cgc gcc gca ttg gcc gcc acg 1728
   His Gly Gly Arg Ala Met Phe Ala Ile Arg Ala Ala Leu Ala Ala Thr
   480 485 490
   atg tct cct gtg tgg ggc gtg tac tcc gga tat gag ctt ttt gag cat 1776
   Met Ser Pro Val Trp Gly Val Tyr Ser Gly Tyr Glu Leu Phe Glu His
   495 500 505 510
   gag gca gtg aag cct ggc tcg gag gag tac ttg gat tct gag aag tac 1824
   Glu Ala Val Lys Pro Gly Ser Glu Glu Tyr Leu Asp Ser Glu Lys Tyr
                                               520                     525
   gaa ctg cgt ccc cgc gat ttc aag ggt gct ctg gaa cgt ggc gat tct      1872
   Glu Leu Arg Pro Arg Asp Phe Lys Gly Ala Leu Glu Arg Gly Asp Ser
               530                 535                 540
   ctc gag gat tac atc gct ctg ctc aac cag atc cgt cgc gcg aac cct      1920
   Leu Glu Asp Tyr Ile Ala Leu Leu Asn Gln Ile Arg Arg Ala Asn Pro
           545                 550                 555
   gcc ttg cag cag cta cgc aac atc cac ttc cac gaa gcg gac aat gat      1968
   Ala Leu Gln Gln Leu Arg Asn Ile His Phe His Glu Ala Asp Asn Asp
       560                 565                 570
   cag atc atc gcc tac tcc aag gtt gat gct ttg acc gga aat acc gtg      2016
   Gln Ile Ile Ala Tyr Ser Lys Val Asp Ala Leu Thr Gly Asn Thr Val
   575                 580                 585                 590
   ttg att gtg gtc aac ttg gat cca cgt agt gct cgt gag gct act gtt      2064
   Leu Ile Val Val Asn Leu Asp Pro Arg Ser Ala Arg Glu Ala Thr Val
                   595                 600                 605
   cgc ctt gat ctt gga gcg ctt ggc tta gaa gcg ggt gca cag ttt gag      2112
   Arg Leu Asp Leu Gly Ala Leu Gly Leu Glu Ala Gly Ala Gln Phe Glu
               610                 615                 620
   gtg cgc gat gcg atc acc gac tcc cgt tac ctg tgg tca gag acg aac      2160
   Val Arg Asp Ala Ile Thr Asp Ser Arg Tyr Leu Trp Ser Glu Thr Asn
           625                 630                 635
   ttt gtc cgc ctc gag ccc cta cgc gat gtc gcc cac atc ttt gtt ctt      2208
   Phe Val Arg Leu Glu Pro Leu Arg Asp Val Ala His Ile Phe Val Leu
       640                 645                 650
   cct gaa ctt cca gcg tct cgc cgt gaa cgt ctt gca tgg cgc gaa atc      2256
   Pro Glu Leu Pro Ala Ser Arg Arg Glu Arg Leu Ala Trp Arg Glu Ile
   655                 660                 665                 670
   aaa acc tac cgc gcg taatttccca tctctgtacc ttctatcaag gattatcatc      2311
   Lys Thr Tyr Arg Ala
                   675
   atgaccgttg accccgcgag ccacatcacc atccctgagg cagatctggc ccgcctgcgc    2371
   cactgcaacc atcacgatcc tcacgggttt tatggttggc atgagaccga agctggttcg    2431
   gttatccgca cgcgccagat cggcgcaacg caggttaatt tgcttatcga cgacacctcc    2491
   cacgtcatga ccccaatcgg cgacgacatt ttcgcaattg acttaggtca ccgcgagcgg    2551
   gctga                                                                2556
<210> 2
   <211> 675
   <212> PRT
   <213> Corymebacterium glutamicum JCM1321
<400> 2
<210> 3
   <211> 23
   <212> DNA
   <213> Artificial sequence
<220>
   <223> Oligonucleotide as PCR sense primer designed from N-terminal amino acid
   sequence of Corynebacterium glutamicum maltose-1-phosphate production enzyme.
<400> 3
   gayggnaayc cngcnaargc ngt 23
<210> 4
   <211> 23
   <212> DNA
   <213> Artificial sequence
<220>
   <223> Oligonucleotide as PCR sense primer designed from N-terminal amino acid
   sequence of Corynebacterium glutamicum maltose-1-phosphate production enzyme.
<400> 4
   gcngtngtng gngarathgt ncc 23
<210> 5
   <211> 30
   <212> DNA
   <213> Artificial sequence
<220>
   <223> Oligonucleotide as PCR anti-sense primer designed from PCR fragment.
<400> 5
   tgaataggct cagccgccac tgaagaatcc 30
<210> 6
   <211> 28
   <212> DNA
   <213> Artificial sequence
<220>
   <223> Oligonucleotide as PCR sense primer designed from PCR fragment.
<400> 6
   tcagatcatc gcctactcca aggttgat 28
<210> 7
   <211> 24
   <212> DNA
   <213> Artificial sequence
<220>
   <223> Oligonucleotide as PCR anti-sense primer designed from PCR fragment.
<400> 7
   acgccacaca atagccgaga cagg 24
<210> 8
   <211> 30
   <212> DNA
   <213> Artificial sequence
<220>
   <223> Oligonucleotide as PCR sense primer designed from PCR fragment.
<400> 8
   ctgtggtcag agacgaactt tgtccgcctc 30
<210> 9
   <211> 27
   <212> DNA
   <213> Artificial sequence
<220>
   <223> Oligonucleotide as PCR sense primer designed from PCR fragment.
<400> 9
   ggagagattc gtcattgagt tcactcg 27
<210> 10
   <211> 26
   <212> DNA
   <213> Artificial sequence
<220>
   <223> Oligonucleotide as PCR anti-sense primer designed from PCR fragment
<400> 10
   tcagcccgct cgcggtgacc taagtc 26

## Description

### Technical Field

The present invention relates to a novel enzyme according to Seq ID No:2 capable of producing maltose-1-phosphate, and to a method for producing maltose-1-phosphate as defined in the main claim.

### Background Art

Maltose-1-phosphate (hereinafter may be abbreviated as "M-1-P") is found in spinach chloroplast (see, for example, Non-Patent Document 1) and *Mycobacterium* cells (see, for example, Non-Patent Document 2). M-1-P has been reported to participate in inhibitory mechanism of cell adhesion (see, for example, Non-Patent Documents 3, 4, and 5), and has found utility in, for example, foods, cosmetic products, pH buffers, reagents for research, and starting materials of substrates for enzyme.

A known method for producing M-1-P employs M-1-P-producing enzyme, and specific examples thereof which have heretofore been reported include maltose kinase of *Actinoplanes missouriensis* (see, for example, Non-Patent Document 6) and maltose synthase contained in spinach (see, for example, Non-Patent Document 7). However, the former enzyme requires ATP for proceeding reaction of maltose and phosphoric acid with a substrate, and the latter requires two glucose-1-phosphate molecules for a substrate, and in consideration of high substrate cost, methods employing these enzymes are not practical in industrial production of M-1-P.
Non-Patent Document 1: FEBS Letters 1976, 61 (2): 192-3;
Non-Patent Document 2: Enzyme Microb. Technol. 1995, 17,140-146;
Non-Patent Document 3: Atherroscleosis 1998, 136 (2): 297-303;
Non-Patent Document 4: Acta Histochem. 1997, 99 (4): 401-410;
Non-Patent Document 5: J. Immunol. 1989, 143 (11): 3666-3672;
Non-Patent Document 6: Arch Microbiol. 2003, 180 (4): 233-239;
Non-Patent Document 7: Planta. 1982, 154: 87-93.

### Disclosure of the Invention

The present invention provides a method for producing maltose-1-phosphate, characterized by causing a maltose-1-phosphate-producing enzyme, having a homology of 90% or more to an amino acid sequence according to SEQ ID No. 2, which is capable of producing maltose-1-phosphate from a phosphoric acid or a salt thereof and an oligosaccharide or polysaccharide containing an α-1,4-glycosidic bond and having a glucose polymerization degree of 5 or more, to act on a phosphoric acid or a salt thereof and an oligosaccharide or polysaccharide having a glucose polymerization degree of 5 or more and containing an α-1,4-glycosidic bond.

In one embodiment of said method, the maltose-1-phosphate-producing enzyme has the following enzymatic characteristics;
1) Substrate specificity: in the presence of a phosphoric acid or a salt thereof, effectively acting on oligosaccharide or polysaccharide containing an α-1,4-glucosidic bond and having a glucose polymerization degree of 6 or more, or a degraded product thereof, thereby producing maltose-1-phosphate; weakly acting on an oligosaccharide having a glucose polymerization degree of 5; and exhibiting substantially no action on an oligosaccharide having a polymerization degree of 2 to 4,
2) Molecular weight: about 75 kDa (SDS-PAGE),
3) Optimum pH: 6.5 to 8.0, and
4) Optimum temperature: 35 to 50°C.

In a particular embodiment, the maltose-1-phosphate-producing enzyme has an amino acid sequence represented by SEQ ID NO: 2.

The invention also provides a method as described above, wherein the maltose-1-phosphate-producing enzyme is encoded by a gene having the following DNA (a) or (b);
(a) a DNA which has a nucleotide sequence represented by SEQ ID NO: 1 which encodes a protein capable of producing maltose-1-phosphate from a phosphoric acid or a salt thereof and an oligosaccharide or polysaccharide containing an α-1,4-glycosidic bond and having a glucose polymerization degree of 5 or more;
(b) a DNA which is capable of hybridizing a DNA fragment having the nucleotide sequence of (a) under stringent conditions and which encodes a protein capable of producing maltose-1-phosphate from a phosphoric acid or a salt thereof and an oligosaccharide or polysaccharide containing an α-1,4-glycosidic bond and having a glucose polymerization degree of 5 or more.
   Furthermore, the invention provides a method for producing maltose-1-phosphate as described above, comprising culturing a microorganism capable of producing the maltose-1-phosphate-producing enzyme in a medium containing 1 mM or more of a phosphoric acid or a salt thereof and an oligosaccharide or polysaccharide having a glucose polymerization degree of 5 or more and containing an α-1,4-glycosidic bond, and collecting maltose-1-phosphate produced and accumulated in the medium.
   In one embodiment of the method of the invention as described above, the microorganism contains a recombinant vector containing a gene encoding a maltose-1-phosphate-producing enzyme having the following DNA (a) or (b);
   (a) a DNA which has a nucleotide sequence represented by SEQ ID NO: 1 which encodes a protein capable of producing maltose-1-phosphate from a phosphoric acid or a salt thereof and an oligosaccharide or polysaccharide containing an α-1,4-glycosidic bond and having a glucose polymerization degree of 5 or more;
   (b) a DNA which is capable of hybridizing a DNA fragment having the nucleotide sequence of (a) under stringent conditions and which encodes a protein capable of producing maltose-1-phosphate from a phosphoric acid or a salt thereof and an oligosaccharide or polysaccharide containing an α-1,4-glycosidic bond and having a glucose polymerization degree of 5 or more. According to the invention, the said microorganism can be a bacterium belonging to the family *Corynebacterium.*

In certain embodiments of the method according to any aspect of the invention the concentration of a phosphoric acid or a salt thereof is 50 mM to 2,000 mM.

The invention also provides a maltose-1-phosphate-producing enzyme having the amino acid sequence according to SEQ ID No. 2, which is capable of producing maltose-1-phosphate from a phosphoric acid or a salt thereof and an oligosaccharide or polysaccharide containing an α-1,4-glycosidic bond and having a glucose polymerization degree of 5 or more; or a gene encoding the said protein.

In specific embodiments, the gene is represented by SEQ ID NO: 1, which encodes a protein capable of producing maltose-1-phosphate from a phosphoric acid or a salt thereof and an oligosaccharide or polysaccharide containing an α-1,4-glycosidic bond and having a glucose polymerization degree of 5 or more.

The invention also provides a recombinant vector containing a gene as defined above.

In a further aspect, the invention provides a transformant containing a recombinant vector of the invention, wherein in some aspects of the invention the host is a microorganism.

### Brief Description of the Drawings

Fig. 1 shows a pH vs. activity curve of the enzyme of the present invention.
Fig. 2 shows a temperature vs. activity curve of the enzyme of the present invention.

### Best Modes for Carrying Out the Invention

The present invention relates to provision of an M-1-P-producing enzyme according to Seq ID No:2 capable of producing a large amount of M-1-P through use of, as a starting material, an oligosaccharide or polysaccharide containing an α-1,4-glycosidic bond and having a glucose polymerization degree of 5 or more. The present invention also relates to a method for producing an M-1-P-producing enzyme capable of producing a large amount of M-1-P through use of the oligosaccharide or polysaccharide as a starting material. The present invention further relates to a method for producing M-1-P, the method enabling, at low cost, production of a large amount of M-1-P through use of the oligosaccharide or polysaccharide as a starting material.

The present inventors have searched for natural microorganisms and enzymes which can produce M-1-P in a medium, and, as a result, have discovered a novel M-1-P-producing enzyme according to Seq ID No:2 capable of producing M-1-P from a phosphoric acid or a salt thereof and an oligosaccharide or polysaccharide having a glucose polymerization degree of 5 or more and containing an α-1,4-glycosidic bond. The present inventors have also found that, by culturing a microorganism capable of producing the enzyme in a phosphate-rich medium, the enzyme is produced in the supernatant of the culture broth at high yield. The present inventors have also found that, by culturing a bacterium belonging to the family *Corynebacterium* in the presence of a sugar and a phosphoric acid or a salt thereof at high concentration, highly concentrated M-1-P is directly obtained in the medium in a large amount.

Use of the M-1-P-producing enzyme according to Seq ID No:2 of the present invention (hereinafter may be referred to as "the enzyme of the present invention") enables production of a large amount of M-1-P with ease, employing as a substrate an inexpensive oligosaccharide or polysaccharide containing an α-1,4-glycosidic bond. According to the present invention, an M-1-P-producing enzyme can be produced in an effective and convenient manner, through a very simple process including culturing a microorganism, separating the culture broth supernatant (removal of cells), and purification. According to the method of the present invention, M-1-P can be produced directly in a medium at high concentration, enabling production of a large amount of M-1-P at low cost and without cumbersome processes.

The enzyme of the present invention may be prepared by, for example, culturing a microorganism capable of producing the M-1-P-producing enzyme of the present invention in a medium containing a sugar, preferably an oligosaccharide or polysaccharide having a glucose polymerization degree of 5 or more and containing an α-1,4-glycosidic bond, and collecting the enzyme from the cultured product. Alternatively, the enzyme of the present invention may be prepared by artificially expressing a gene encoding the enzyme of the present invention, thereby producing a polypeptide having M-1-P-producing enzyme activity, and collecting the same.

Examples of the microorganism capable of producing the enzyme of the present invention include bacteria belonging to the family *Corynebacterium such* as *Corynebacterium* sp. JCM1300, *Corynebacterium flavescens, Corynebacterium glutamicum, Corynebacterium hoagii, Corynebacterium vitaeruminis, Corynebacterium pilosum, Corynebacterium amycolatum, Corynebacterium matruchoti, Corynebacterium* minutissimum, *Corynebacterium* striatum, and *Corynebacterium callunae.* Of these, *Corynebacterium* sp. JCM1300, *Corynehacterium flavescens* JCM1317, *Corynebacterium glutamicum* JCM1318, *Corynebacterium hoagii* JCM1319,' *Corynebacterium glutamicum* JCM1321, *Corynebacterium vitaeruminis* JCM1323, *Corynebacterium pilosum* JCM3714, *Corynebacterium amycolatum* JCM7447, *Corynebacterium matruchotii* JCM9386, *Corynebacterium* minutissimum JCM9387, *Corynebacterium* striatum JCM9390, and *Corynebacterium callunae* IFO15359 are preferred. From the viewpoint of high M-1-P productivity, *Corynebacterium sp.* JCM1300, *Corynebacterium hoagii* JCM1319, *Corynebacterium glutamicum* JCM1321, and *Corynebacterium callunae* IFO15359 are particularly preferred. Alternatively, there may be employed a transformant, described below, containing a recombinant vector containing a gene of the enzyme of the present invention.

Examples of sugar (carbon source) incorporated into the medium includes monosaccharide, disaccharide, oligosaccharide, and polysaccharide. The sugars may be employed in combination of two or more species. Of these, preferred species include an oligosaccharide or polysaccharide having a glucose polymerization degree of 5 or more and containing an α-1,4-glycosidic bond. Examples of the oligosaccharide or polysaccharide having a glucose polymerization degree of 5 or more and containing an α-1,4-glycosidic bond include starch, amylose, dextrin, maltose, maltooligosaccharide, amylopectin, glycogen, and starch-degraded products. Among them, starch, dextrin, and maltooligosaccharide, which are inexpensive, are preferred. These sugars may be employed in combination of two or more species. The concentration of the oligosaccharide or polysaccharide in the medium is preferably 1 to 70 mass%, more preferably 10 to 70 mass%, still more preferably 20 to 50 mass%, from the viewpoint of effectiveness.

The above-described culture is preferably performed in a medium further containing a phosphoric acid or a salt thereof. The concentration of a phosphoric acid or a salt thereof is typically 50 mM or more, preferably 50 mM to 2,000 mM, more preferably 100 mM to 1,200 mM, still more preferably 400 to 1,000 mM. When the amount of a phosphoric acid or a salt falls within the above range, an M-1-P-producing enzyme can be effectively produced and accumulated in the culture broth supernatant. Examples of the phosphoric acid or a salt thereof include phosphoric acid, metaphosphoric acid, tripolyphosphoric acid, polyphosphoric acid, diphosphoric acid, polymetaphosphoric acid, and the salt thereof, and examples of the salt include a sodium salt and a potassium salt. Preferred examples of the phosphoric acid salt include monopotassium phosphate, dipotassium phosphate, monosodium phosphate, and disodium phosphate.

No particular limitations are imposed on the medium employed in the present invention, so long as the medium allows growth of a bacterium capable of producing an M-1-P-producing enzyme. There may be employed a liquid medium containing, in addition to the above sugar or polysaccharide, a carbon source other than saccharide, a nitrogen source, metallic minerals, or vitamins. Examples of the carbon source other than sugar include organic acid salts such as acetic acid salts. Examples of the nitrogen source include ammonia; inorganic and organic ammonium salts such as ammonium chloride, ammonium sulfate, ammonium nitrate, ammonium carbonate, ammonium phosphate, and ammonium acetate; nitrogen-containing organic substances such as urea, peptone, meat extract, yeast extract, and casein-hydrolyzed products; and amino acids such as glycine, glutamic acid, alanine, and methionine. Examples of the metallic minerals include sodium chloride, ferrous sulfate, magnesium sulfate, manganese sulfate, zinc sulfate, and calcium carbonate. These compounds may be employed singly or in combination of two or more species in accordance with needs.

The culture is performed under pH and temperature conditions which are appropriately adjusted so that the microorganism can be grown sufficiently. The culture may be performed through shaking culture, anaerobic culture, stationary culture, or culture in a fermentation tank. Alternatively, resting cell reaction or immobilized cell reaction may be employed.

Collection of the M-1-P-producing enzyme from the cultured product may be performed through a known method. For example, if necessary, the cultured product is pretreated to bacteriolysis through treatment with ultrasound or with surfactant, the cultured product is subjected to separate and remove the cells through centrifugation or filtration.
And the supernatant is subjected to ultrafiltration, salting out, solvent precipitation, ion exchange, hydrophobic chromatography, gel filtration, drying, or a similar process, or a combination of such processes, to thereby concentrate the enzyme.

In these steps, the enzyme is caused to precipitate through salting out employing, for example, ammonium sulfate or through solvent precipitation employing, for example, cold acetone. Subsequently, the precipitated product is subjected to centrifugation or desalting. Thus, a freeze-dried powder or spray-dried powder can be obtained. In the desalting step, dialysis, gel filtration through use of, for example, Sephadex G-10 (Pharmacia Biotech), ultrafiltration, or a similar process is employed. The thus-obtained enzyme solution or dried powder may be employed without further treatment, or may further be subjected to crystallization or granulation through a known method.

As described above, when culture is performed in a medium containing a phosphoric acid or a salt thereof at high concentration, the enzyme of the present invention is extracellularly produced, and can readily be obtained through collection from the cultured product. In addition, since the enzyme of the present invention is extracellularly produced, an immobilized enzyme can be produced without cumbersome processes.

Thus, according to the method of the present invention employing a phosphoric acid or a salt thereof at high concentration, the M-1-P-producing enzyme can be produced outside the cells directly, and can thus be obtained without intricate processes such as cell pulverization. In addition, the medium is merely supplemented with a phosphoric acid, which is a compound having a low molecular weight, burden to the purification steps are considerably reduced, and a high-purity product of the enzyme can readily be prepared.

According to the method, no special treatment is needed to produce M-1-P- in the culture broth. In addition, a glycoside can be obtained with no special procedure, by directly causing reaction of a compound serving as an aglycon with M-1-P in the culture broth.

The thus-produced enzyme of the present invention is a novel M-1-P-producing enzyme which is capable of producing M-1-P from a phosphoric acid or a salt thereof and an oligosaccharide or polysaccharide having a glucose polymerization degree of 5 or more and containing an α-1,4-glycosidic bond. The enzymatic properties of the enzyme will next be described. Measurement of the enzyme activity was performed through use of, as substrates, 2% dextrin MAX 1000 (Matsutani Chemical) and 1M phosphate buffer (pH 7) in the following manner: a suitably diluted enzyme was added to the substrates, the mixture was incubated for 1 hour at 37°C, and the produced M-1-P was quantified through HPLC (see Example 1).

### 1) Action

The enzyme of the present invention produces M-1-P from a phosphoric acid or a salt thereof, and an oligosaccharide or polysaccharide having a glucose polymerization degree of 5 or more and containing an α-1,4-glycosidic bond.

The enzyme of the present invention exhibits phosphorylase activity; i.e., the enzyme acts, in the presence of a phosphoric acid or a salt thereof, on an oligosaccharide or polysaccharide having a glucose polymerization degree of 5 or more and containing an α-1,4-glycosidic bond, and recognizes a maltose unit thereof, to thereby catalyze phosphorolysis of the oligosaccharide or polysaccharide. No phosphorylase has been reported, except for a phosphorylase which recognizes glucose or other monosaccharides to thereby produce glucose-1-phosphate or other products, and nothing has been known in relation to an enzyme which recognizes disaccharide to produce disaccharide phosphate.

In the absence of a phosphoric acid, the enzyme of the present invention acts on a maltooligosaccharide and transfers per unit of maltose. That is, when the enzyme of the present invention acts on maltohexaose (DP=6), a maltooligosaccharide (DP=4, DP=6, DP=8, DP=10, etc.) is produced, and when the enzyme of the present invention acts on maltoheptaose (DP=7), maltooligosaccharide (DP=5, DP=7, DP=9, DP=11, etc.) is produced. Thus, the enzyme of the present invention has activity of acting on maltopentaose or higher oligosaccharide to cause maltose-unit transfer. Although a maltosyltransferase has already been reported in Eur. J. Biochem. (1998) 250, 1050-1058, no report describes that the maltosyltransferase produces M-1-P. Moreover, the existing maltosyltransferase acts on maltotriose (DP=3) or other low molecules, and, when the existing maltosyltransferase is caused to act on an oligosaccharide of DP = 3 or higher, a low-polymerized oligosaccharide of DP = 1 to 3 having a low molecular weight is produced. On the contrary, the enzyme of the present invention exhibits substantially no action on maltooligosaccharide of DP = 3 or 4, and weakly acts on maltooligosaccharide of DP = 5. In addition, even when the enzyme of the present invention acts on maltooligosaccharide of DP = 5 or higher, substantially no oligosaccharide of low polymerization.degree (DP = 1 to 3) is produced. Therefore, the enzyme of the present invention is different from the existing maltosyltransferase.

Thus, the enzyme of the present invention is a novel enzyme having both phosphorylase activity (i.e., capable of acting on an oligosaccharide or polysaccharide having a glucose polymerization degree of 5 or more and containing an α-1,4-glycosidic bond and recognizing a maltose unit to thereby cause phosphorolysis of the oligosaccharide or polysaccharide) and maltose transfer activity (i.e., capable of extending by unit of maltose). Therefore, the enzyme of the present invention may be named, for example, maltodextrin-maltosylphosphorylase, maltodextrin : orthophosphoric acid-α-1-maltosyltransferase, or maltosyltransferase.

Examples of the oligosaccharide or polysaccharide having a glucose polymerization degree of 5 or more and containing an α-1,4-glycosidic bond (i.e., a substrate of the enzyme of the present invention) include starch, amylose, dextrin, maltose, maltooligosaccharide, amylopectin, glycogen, and starch-degraded products.

Examples of the phosphoric acid or a salt thereof include phosphoric acid, metaphosphoric acid, tripolyphosphoric acid, polyphosphoric acid, diphosphoric acid, polymetaphosphoric acid, and a salt thereof. The salt is preferably a sodium salt or potassium salt. Particularly preferred examples of the phosphoric acid salt include monopbtassium phosphate, dipotassium phosphate, monosodium phosphate, and disodium phosphate.

No particular limitations are imposed on the concentration of the substrates. However, the concentration of a phosphoric acid or a salt thereof is preferably 50 mM to 2,000 mM, more preferably 100 mM to 1,200 mM, even more preferably 400 to 1,000 mM. The concentration of the oligosaccharide or polysaccharide having a glucose polymerization degree of 5 or more and containing an α-1,4-glycosidic bond is preferably 1 to 70 mass%.

### 2) Substrate specificity

The enzyme of the present invention effectively acts on, in the presence of a phosphoric acid or a salt thereof, an oligosaccharide or polysaccharide having a glucose polymerization degree of 6 or more and containing an α-1,4-glucosidic bond or a degraded product of the oligosaccharide or polysaccharide to thereby produce M-1-P. The enzyme of the present invention weakly acts on an oligosaccharide having a glucose polymerization degree of 5, and exhibits substantially no action on an oligosaccharide having a polymerization degree of 2 to 4. Examples of the polysaccharide include amylose, amylopectin, glycogen, dextrin, and starch.

### 3) Molecular weight

The enzyme of the present invention has a molecular weight of about 75 kDa as measured through sodium dodecyl sulfate-polyacrylamide gel electrophoresis (SDS-PAGE) (it should be noted that, when SDS-PAGE is employed, measurement data may fluctuate by ± 5 kDa or thereabouts, depending on the SDS-PAGE conditions).

### 4) Optimum pH and active pH

As substrates, 2% maltoheptaose (G7: Seikagaku Corporation) and 700mM phosphate buffers at every pH from 5.5 to 8.5 are employed. The purified enzyme is added to the substrates so as to attain a concentration of 0.28 U/mL(see Example 1), and the mixture is allowed to react for 1 hour at 37°C, followed by measurement of enzyme activity. The enzyme of the present invention exhibits an optimum pH ranging from about 6.5 to about 8.0. In this range, the enzyme exhibits 80% or more of the activity measured at pH 7.5 (the maximum activity). At a pH of 5.5 to 8.5, the enzyme exhibits 50% or more of the activity measured at pH 7.5.

### 5) Optimum temperature and active temperature

As substrates, 1M potassium phosphate buffer (pH 7) and 2% maltoheptaose (G7: Seikagaku Corporation) are employed. The purified enzyme is added to the substrate so as to attain a concentration of 0.168 U/mL, and the mixture is allowed to react for 1 hour at a temperature of 30°C to 70°C. The reaction mixture is treated for 10 minutes at 95°C for deactivation of the enzyme. The reaction mixture is diluted 101-fold, followed by measurement of enzyme activity. The enzyme of the present invention exhibits an optimum temperature of 35 to 50°C. In this range, the enzyme exhibits 80% or more of the activity measured at 40°C (the maximum activity). At a temperature of 30°C to 55°C, the enzyme exhibits 30% or more of the activity measured at 40°C.

Next will be described the protein of the present invention and a gene encoding the protein.

The protein of the present invention is capable of producing M-1-P, as described above, and defined by an amino acid sequence represented by SEQ ID NO: 2.

In the context of the method of the present invention, the maltose-1-phosphate-producing enzyme is characterized by having a homology of 90% or more to an amino acid sequence according to SEQ ID No. 2, which enzyme is capable of producing maltose-1-phosphate from a phosphoric acid or a salt thereof and an oligosaccharide or polysaccharide containing an α-1,4-glycosidic bond and having a glucose polymerization degree of 5 or more.

The 675 amino acid residues represented by SEQ ID NO: 2 were subjected to BLASTP homology search of the NCBI database, which revealed that the sequence exhibits 99% to 38% homology with proteins originating from microorganisms such as *Corynebacterium glutamicum* ATCC13032 (99%), *Corynebacterium efficiens* YS-314 (84%), *Corynebacteriumd iphtheriae* (66%), and *Mycobacterium smegmatis* (60%) (see Example 7). These proteins are classified into the Family 13 of Glycosyl Hydrolase in the saccharide-related enzyme database CAZy, and deduced to be glycosidase, amylase, glucanase, or the like. However, the deduction is merely based on the genomic sequence of these proteins. Genes of the proteins have never been isolated, and expression of the genes to produce the proteins has never been examined. Thus, nothing has been known in relation to the function. Comparison of the amino acid sequences in terms of the alignment reveals highly conserved regions of the 342nd to 352nd amino acid residues (Ara-Glu-Asn-Pro-Pro-Lys-Lys(or Arg)-Tyr-Gln(or Glu)-Asp-Ile) [see the 342nd to 352nd of SEQ ID NO: 2] and the 383rd to 392nd amino acid residues (Phe-Arg-Val(or Ile)-Asp-Asn-Pro-His-Thr-Lys-Pro) [see the 383rd to 392nd of SEQ ID NO: 2] from the N-terminus of the amino acid sequence of the M-1-P-producing enzyme of the present invention.
Therefore, the protein for use in the method of the present invention includes, as preferred examples, proteins which exhibit 90% or more, further more preferably 95% or more homology with SEQ ID NO: 2, which is capable of producing M-1-P, and which has an amino acid sequence represented by Ara-Glu-Asn-Pro-Pro-Lys-Lys(or Arg)-Tyr-Gln(or Glu)-Asp-Ile and/or Phe-Arg-Val(or Ile)-Asp-Asn-Pro-His-Thr-Lys-Pro.

No particular limitations are imposed on the gene of the M-1-P-producing enzyme of the present invention, so long as the gene encodes any of the above proteins. However, preferred are genes containing a nucleotide sequence represented by SEQ ID NO: 1 and genes containing DNA which are capable of hybridizing with a DNA fragment having a nucleotide sequence represented by SEQ ID NO: 1 under stringent conditions.

Examples of the "under stringent conditions" include a condition described in Molecular cloning - a Laboratory Manual, 2nd edition (Sambrook et al., 1989). For example, hybridization is performed by adding a DNA fragment and a probe to a solution containing 100 mg/mL of herring sperm DNA and 6xSSC (composition of 1×SSC; 0.15M sodium chloride, 0.015M sodium citrate, pH 7.0), 0.5% SDS, and 5×Denhart, and incubating the mixture for 8 to 16 hours at 65°C.
The enzyme gene of the present invention encompasses a DNA which exhibits 60% or more homology with the nucleotide sequence represented by SEQ ID NO: 1 and which encodes a protein capable of producing M-1-P. The homology of the sequence is preferably 70% or more, more preferably 80% or more, still more preferably 90% or more, further more preferably 95% or more.

The homology of the amino acid sequence or the nucleotide sequence is calculated through the Lipman-Pearson method (Science, 227, 1435, (1985)). Specifically, analysis is performed through use of a homology analysis (Search homology) program of genetic information processing software Genetyx-Win (Ver.5.1.1; Software Development Co., Ltd.) (Unit size to compare (ktup) = 1).

The enzyme gene of the present invention may be obtained by examining chromosomal DNA of the enzyme-producing microorganism employed in the present invention (such as microorganisms belonging to the family *Corynebacterium* as mentioned above) through a typical DNA cloning technique based on the DNA nucleotide sequence or the amino acid sequence according to the present invention. The examination of chromosomal DNA may be performed through hybridization employing a library, PCR employing the chromosomal DNA as a template, or a modified method of these techniques.

A recombinant vector containing the enzyme gene of the present invention may be prepared by introducing the enzyme gene into a vector which can be replicated and maintained in a host cell, which allows stable expression of the enzyme, and which can stably maintain the gene. Examples of the vector employable when *Escherichia coli* is used as a host include pUC18, pBR322, and pHY300PLK. Examples of the vector employable when *Bacillus subtilis* is used as a host include pUB110, pHSP64 (Sumitomo et al., Biosci. Biotechnol. Biocem., 59, 2172-2175, 1995), and pHY300PLK (Takara Bio Inc.).

Expression of the enzyme gene of the present invention is preferably performed by transferring the enzyme gene of the present invention to a host cell to thereby prepare a transformant, seeding the transformant in a medium containing an assimilable carbon source, an assimilable nitrogen source, and other essential nutrients, and culturing through a routine method. The transformant may be obtained by ligating the enzyme gene of the present invention to an autonomously replicable vector to thereby prepare a recombinant DNA, and introducing the recombinant DNA into a host microorganism which is suitable for expression of the DNA. No particular limitations are imposed on the host bacterium, and examples include Gram-positive bacteria such as bacteria belonging to the family *Bacillus (Bacillus subtilis*), Gram-negative bacteria such as *Escherichia coli,* and fungi such as those belonging to the family *Streptomyces* (actinomycetes), the family *Saccharomyces* (yeast), or the family *Aspergillus* (molds). Collection and purification of the enzyme contained in the culture broth may be performed through a routine method, and the thus-obtained enzyme may be further subjected to lyophilization, spray-drying, or crystallization in accordance with needs.

M-1-P can be produced in a large amount through use of the thus-produced enzyme of the present invention at low cost. Specifically, the enzyme of the present invention is added to a solution containing the substrates; i.e., a phosphoric acid or a salt thereof and an oligosaccharide or polysaccharide having a glucose polymerization degree of 5 or more and containing an α-1,4-glycosidic bond, to thereby cause reaction of the enzyme with the substrate. Alternatively, the enzyme of the present invention is caused to react with the substrates through an immobilized enzyme method; i.e., the enzyme of the present invention is immobilized to a carrier, and the substrates are caused to flow through a column containing the carrier. No particular limitations are imposed on the carrier to which the enzyme is immobilized, so long as the carrier is commonly used. For example, an ion exchange resin or a synthetic absorption resin may be employed.

Alternatively, M-1-P can be obtained by culturing any of the above-mentioned microorganisms which are capable of producing the enzyme of the present invention in a medium containing a sugar and a phosphoric acid or a salt thereof, to thereby yield M-1-P in the culture broth.

Preferred examples of the sugar incorporated into the medium include monosaccharide, disaccharide, oligosaccharide, and polysaccharide which contain glucose as a structural sugar. Among them, more preferred is an oligosaccharide or polysaccharide containing an α-1,4-glycosidic bond, such as starch, amylose, dextrin, maltose, maltooligosaccharide, amylopectin, glycogen, or a starch-degraded product. Of these, starch, dextrin, and maltooligosaccharide, which are inexpensive, are preferred. These sugars may be employed in combination with two or more species.

Concentration of sugar in the medium is preferably 1 to 70 mass%, more preferably 10 to 70 mass%, even more preferably 20 to 50 mass%, from the viewpoint of effectiveness.

Examples of the phosphoric acid or a salt thereof incorporated into the medium include phosphoric acid, metaphosphoric acid, tripolyphosphoric acid, polyphosphoric acid, diphosphoric acid, polymetaphosphoric acid, and salts thereof. The salt is preferably a sodium salt or a potassium salt. Preferred examples of the phosphoric acid salt include monopotassium phosphate, dipotassium phosphate, monosodium phosphate, and disodium phosphate. In the present invention, a phosphoric acid or a salt thereof, or a mixture of a plurality of phosphoric acid salts is preferably employed.

From the viewpoint of effectiveness, the concentration of a phosphoric acid or a salt thereof in the medium is 1 mM or more, and is preferably 1 mM to 2 M, more preferably 50 mM to 1 M.

No particular limitations are imposed on the medium employed, so long as the medium allows growth of a microorganism capable of producing the enzyme of the present invention, and there may be employed, for example, a liquid medium containing, in addition to a sugar and a phosphoric acid or a salt thereof described above, a carbon source, a nitrogen source, metallic minerals, vitamins, etc.

Examples of the carbon source other than sugar include organic acid salts such as acetic acid salts. Examples of the nitrogen source include ammonia; inorganic and organic ammonium salts such as ammonium chloride, ammonium sulfate, ammonium nitrate, ammonium carbonate, ammonium phosphate, and ammonium acetate; nitrogen-containing organic compounds such as urea, peptone, meat extract, yeast extract, and casein-hydrolyzed products; and amino acids such as glycine, glutamic acid, alanine, and methionine. Examples of the metallic minerals include sodium chloride, ferrous sulfate, magnesium sulfate, manganese sulfate, zinc sulfate, and calcium carbonate. These compounds may be employed singly or in combination of two or more species in accordance with needs.

The culture is performed under pH and temperature conditions which are appropriately adjusted so that the microorganism can be grown sufficiently. Typically, the culture is preferably performed for 12 hours to 96 hours at a pH of 4 to 8 and a temperature of 25°C to 40°C. The culture may be performed through stationary culture, shaking culture, or culture in a fermentation tank. In addition, resting cell reaction and immobilized cell reaction may be employed.

Collection of M-1-P contained in the cultured products may be performed through a method known *per se.* According to the method of the present invention, there may be produced, in addition to M-1-P, glucose-1-phosphate or other phosphorylated saccharides. Therefore, after the cells are separated and removed, M-1-P is preferably isolated through, for example, ultrafiltration, reverse osmosis membrane, electrodialysis, ion exchange membrane, ion exchange resin, active carbon, or treatment with a synthetic absorbent, an subsequently through crystallization or salting out, in accordance with needs. The thus-obtained M-1-P may be further purified through ion exchange chromatography or other techniques, in accordance with needs.

As mentioned above, the enzyme which catalyzes production of M-1-P in the method of the present invention for producing M-1-P is a novel enzyme. Therefore, when there is employed a bacterium cell in which a gene region encoding an enzyme that catalyzes production of glucose-1-phosphate or other phosphorylated saccharides is deactivated or eliminated from the genome, M-1-P of interest can be selectively obtained. In particular, bacterium belonging to the family *Corynebacterium* in which a gene encoding extracellular maltodextrin phosphorylase is deactivated is preferably used. The elimination or deactivation of a gene of interest may be performed through a known method (*e.g.*, Mol. Gen. Genet., 223, 268, 1990).

### Examples

### Example 1 Method for determining activity of M-1-P-producing enzyme

Activity of an M-1-P-producing enzyme was determined through the following procedure.

As substrates, 2% dextrin MAX 1000 (Matsutani Chemical) and 1M phosphate buffer (pH 7) were employed. To the substrates, a suitably diluted enzyme was added, and the mixture was incubated for one hour at 37°C, followed by quantification of the produced M-1-P through HPLC. Specifically, quantification of M-1-P was performed through use of a DX500 chromatography system (product of DIONEX Corporation) (column: CarboPac PA1 (4x250 mm), detector: ED40 pulsed amperometric detector, eluent: (solution A) 100mM sodium hydroxide; (solution B) 100mM sodium hydroxide containing 1M sodium acetate). Linear gradient analysis was employed (initial concentration: solution.A 90% : solution B 10%, 0-17 min.: solution A 18.5% : solution B 81.5%). As standard substances, 100mM and 50mM M-1-P products (product of SIGMA) were employed. A peak was observed at about 15.5 minutes. One enzyme unit (1U) is defined as the amount of enzyme that produces 1 µM of M-1-P per minute.

### Example 2 Production of M-1-P-producing enzyme

The strain *Corynebacterium glutamicum* JCM1321 was employed. The bacterium was seeded in an SCD agar medium (Nihon Pharmaceutical Co., Ltd.) and allowed to grow overnight at 30°C. The grown bacterium was subjected to seed culture through the following steps; a medium (50 mL) containing 0.5% yeast extract, 4% amino acid seasoning solution K (Ajinomoto Co., Inc.), 3% maltose-rich syrup (Showa Sangyo Co., Ltd.), and 100mM phosphate buffer (pH 7) was placed in a baffled Erlenmeyer flask; one platinum loop of the grown bacterium was seeded in the medium; and the bacterium was subjected to shaking culture overnight at 30°C, at 210 rpm. Primary culture of the thus-obtained seed bacterium was performed through the following steps; a medium (50 mL) containing 0.5% yeast extract, 1% amino acid seasoning solution K (Ajinomoto Co., Inc.), 0.5% ammonium sulfate, 10% dextrin MAX 1000 (Matsutani Chemical), 10% calcium chloride dihydrate, 200-ppm magnesium sulfate, 25-ppm ferric chloride, and 400mM phosphate buffer (pH 7) was placed in a 160-baffled Erlenmeyer flask; the seed bacterium was seeded in the medium in an amount of 1%; and the bacterium was subjected to shaking culture overnight at 30°C, at 210 rpm.

In the supernatant of the medium, 240 U/L of the M-1-P-producing enzyme was produced.

### Example 3 Isolation and purification of M-1-P-producing enzyme

The primary culture broth (6 L) obtained in Example 2 was subjected to centrifugation, and the supernatant was concentrated through use of an ultrafiltration module ACP-13000 (Asahi Kasei Corporation), followed by dialysis with 10mM phosphate buffer (pH 8). The concentrated dialysate was caused to run through a DEAE-Toyopearl 650M column (Tosoh Corporation; φ5×15 cm) for adsorption, and the column was washed with the same buffer (2 L), followed by elution with 1M sodium chloride (1.5 L), to thereby yield a crude enzyme solution.

The crude enzyme solution was purified by means of a BIO-CAD60 system (Perseptive). Specifically, 1M ammonium sulfate was firstly added to the crude enzyme solution (1,580 mL), and the mixture was caused to run, for adsorption, through a hydrophobic chromatography column POROS PE/M (φ10×100 mm) which had been equilibrated with 1M ammonium sulfate and 50mM phosphate buffer. It was eluted with 150 ml of ammonium sulfate contained in 50mM phosphate buffer (pH 8) with gradient of 1,000 mM to 360 mM, and then 375 mL of ammonia sulfate contained in 50mM phosphate buffer (pH 8) with gradient of 360 mM to 0 mM at flow rate of 12 mL/minute. A fraction corresponding to about 360mM ammonium sulfate was found to exhibit a peak attributed to the M-1-P-producing enzyme. The active fraction was dialyzed with 10mM phosphate buffer (pH 8), thereby yielding 74 mL of an enzyme solution.

The dialyzed enzyme solution was further caused to run, for adsorption, through an anion exchange column POROS HQ/M (φ10×100 mm) which had been equilibrated with 20mM phosphate buffer (pH 8). It was eluted with 450 mL of sodium chloride in 20mM phosphate buffer (pH 8) with gradient of 0 to 50 mM was at flow rate of 12 mL/minute. Directly following the rise of sodium chloride concentration, an active fraction was obtained. The active fraction was dialyzed with 10mM phosphate buffer (pH 8), thereby yielding 9.3 mL of an enzyme solution.

Further, the enzyme solution was applied to an anion exchange column POROS HQ/M (φ10×100 mm) in a manner similar to that described above. Directly following the rise of sodium chloride concentration, a peak which was believed to be attributed to the M-1-P-producing enzyme was detected.

The peak-top fraction was concentrated through use of a Centriprep YM-3 (Millipore) so as to attain a volume of 0.6 mL (i.e., the fraction was concentrated 10-fold). The concentrate was subjected to SDS-PAGE. A substantially single band was detected. The molecular weight was estimated to be 75 kDa. The amino acid sequence of an end of the amino moiety of the sample was determined to be Gly-Arg-Leu-Gly-Ile-Asp-Asp-Val-Arg-Pro-Arg-Ile-Leu-Asp-Gly-Asn-Pro-Ala-Lys-Ala-Val-Val-Gly-Glu-Ile-Val-Pro-Val-Ser-Ala-Ile-Val-Trp-Arg-Glu (the 3rd to 37th of SEQ ID NO: 2).

### Example 4 Intracellular or extracellular activity of M-1-P-producing enzyme

The strains employed were *Corynebacterium glutamicum* JCM1318, *Corynebacterium hoagii* JCM1319, *Corynebacterium glutamicum* JCM1321, *Corynebacterium vitaeruminis* JCM1323, and *Corynebacterium callunae* IFO15359. Each of the bacteria was seeded in an SCD agar plate (Nihon Pharmaceutical Co., Ltd.) and cultured overnight at 30°C. Seed culture was performed through the following steps; 0.67% Yeast Nitrogen Base (Difco) (10 mL) was placed in a large test tube, and one platinum loop of the bacteria was seeded, followed by shaking-culture overnight at 30°C, at 250 rpm.

Primary culture was performed through shaking culture for 6 days at 35°C, at 250 rpm in a medium containing 0.67% Yeast Nitrogen Base (Difco) and 10% dextrin MAX 1000 (Matsutani Chemical) supplemented with or without 400mM potassium phosphate buffer (pH 7.0).

The culture broth (1 mL) was subjected to centrifugation, to thereby separate the supernatant from cells. Extracellular activity of M1P-producing enzyme was determined through use of the supernatant without further treatment. Intracellular activity of M1P-producing enzyme was determined by use of the cells through the following steps; the thus-collected cells were suspended in 50mM potassium phosphate buffer (pH 7.0) (an amount equal to that of the culture broth); the suspension was subjected to centrifugation thereby separating and washing the cells; the resultant cells were suspended in 50mM potassium phosphate buffer (pH 7.0) (an amount equal to that of the culture broth); toluene (50 µL) was added to the suspension; the mixture was vigorously stirred for lysis of the cells to thereby yield an enzyme solution for measurement of activity; and the activity was measured in accordance with the method employed in Example 1. The results are shown in Table 1.

**Table 1**

| | | Without phosphate | | With 400mM phosphate | |
|---|---|---|---|---|---|
| | | Intracellular | Extracellular | Intracellular | Extracellular |
| *Corynebacterium callunae* | IFO15359 | 20.37 | - | 631.33 | 326.58 |
| *Corynebacterium glutamicum* | JCM1321 | 10.02 | - | 177.88 | 485.21 |
| *Corynebacterium vitaeruminis* | JCM1323 | 5.55 | - | 281.25 | 40.60 |
| *Corynebacterium hoagii* | JCM1319 | 4.46 | - | 293.57 | 211.84 |
| *Corynebacterium glutamicum* | JCM1318 | 4.33 | - | 228.26 | 208.81 |

The results indicate the following. When no phosphate is added, the enzyme exhibits activity only inside cells. When the bacterium is cultured in the presence of high concentration of phosphate, the M-1-P-producing enzyme activity is improved, and M-1-P-producing enzyme produced inside cell are released to the culture broth.

### Example 5 Optimum pH and active pH

As substrates, 2% maltoheptaose (G7: Seikagaku Corporation) and 700mM phosphate buffers of each pH from 5.5 to 8.5 were employed. The purified enzyme was added to the substrates so as to attain a concentration of 0.28 U/mL, and the mixture was allowed to react for 1 hour at 37°C. The reaction mixture was treated for 10 minutes at 95°C to stop the reaction, and the mixture was diluted 101-fold, followed by quantification of M-1-P through HPLC in a manner similar to that employed in Example 1.

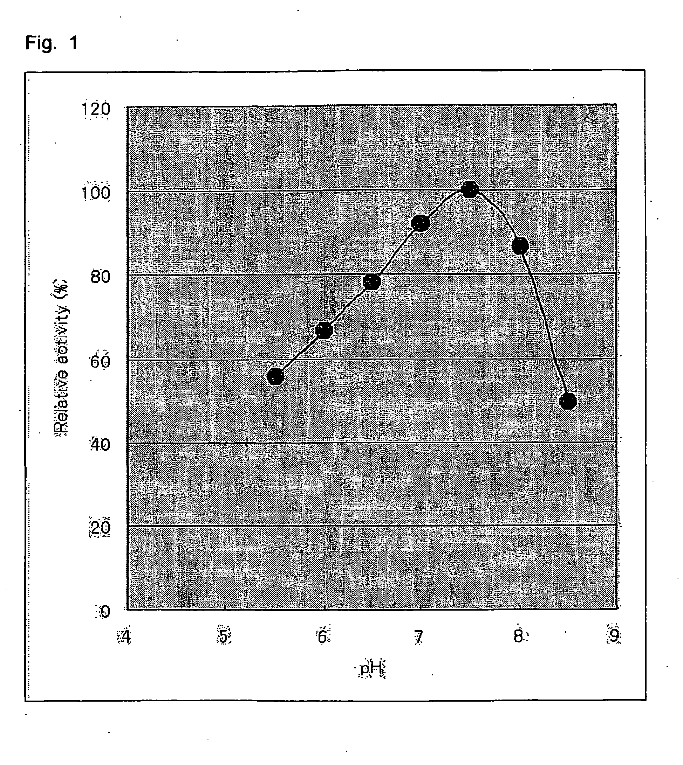

The results are shown in Fig. 1. The optimum pH was found to be about 6.5 to about 8.0. In this range, the enzyme exhibits 80% or more of the activity measured at pH 7.5 (the maximum activity). At a pH ranging from 5.5 to 8.5, the enzyme exhibits 50% or more of the activity measured at pH 7.5, revealing that the enzyme is active in a wide pH range.

### Example 6 Optimum temperature and active temperature

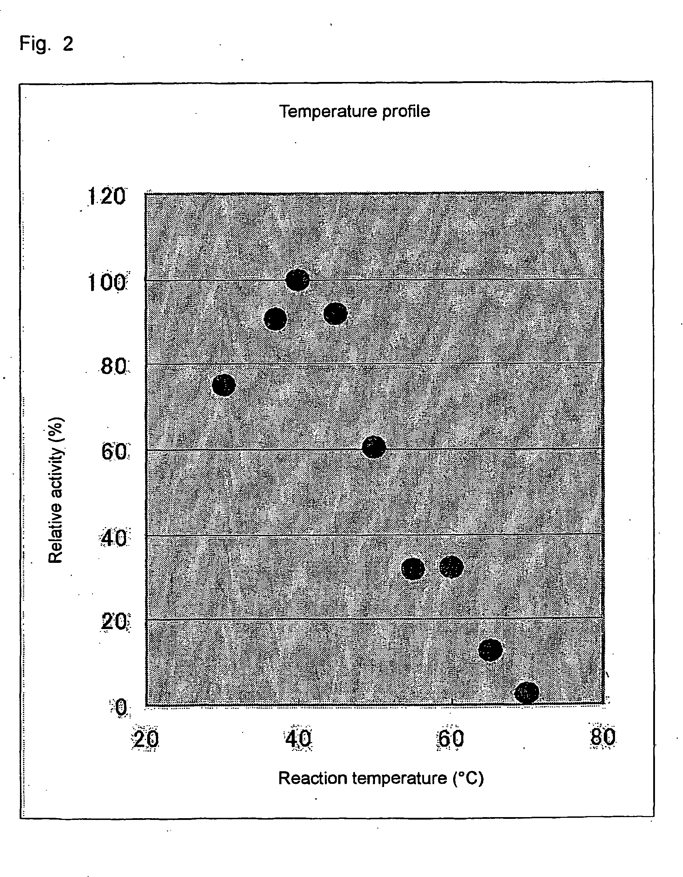

As substrates, 1M potassium phosphate buffer (pH 7) and 2% maltoheptaose (G7: Seikagaku Corporation) were employed. The purified enzyme was added to the substrates so as to attain a concentration of 0.168 U/mL, and the mixture was allowed to react for 1 hour at a temperature of 30°C to 70°C. The reaction mixture was treated for 10 minutes at 95°C for deactivation of the enzyme. The reaction mixture was diluted 101-fold, followed by quantification of M-1-P through HPLC in a manner similar to that described in Example 1. The results are shown in Fig. 2. The optimum temperature was found to be 35°C to 50°C. In the range, the enzyme exhibits 60% or more of the activity measured at 40°C (the maximum activity). In a wide temperature range (30°C to 55°C), the enzyme exhibits 30% or more of the activity measured at 40°C.

### Example 7. Substrate specificity

### (1) Production of M-1-P from dextrin and phosphate

To 2.5% Dextrin MAX 1000 (Matsutani Chemical) and 250mM phosphate buffer (pH 7), 0.16 U/mL M-1-P-producing enzyme was added, and the mixture was allowed to react for 15 hours at 37°C. In a manner similar to that described in Example 1, M-1-P was quantified. The enzyme was found to produce 145µM M-1-P.

### (2) Production of M-1-P from phosphate and different maltooligosaccharides

To 250mM Phosphate buffer (pH 7) and each of substrates having different chain lengths (glucose, maltose, maltotriose, maltotetraose, maltopentaose, maltohexaose (Seikagaku Corporation)) (2.5%), 0.16 U/mL M-1-P-producing enzyme was added, and the mixture was allowed to react for 15 hours at 37°C. In a manner similar to that described in Example 1, M-1-P was quantified. The results are shown in Table 2.

**Table 2**

| Substrate | Amount of maltose-1-phosphate produced (µM) |
|---|---|
| Glucose | ND |
| Maltose | ND |
| Maltotriose | ND |
| Maltotetraose | ND |
| Maltopentaose | 1.04 |
| Maltohexaose | 49.6 |
| Maltoheptaose | 96.1 |

### (3) Maltose transfer reaction

0.16 U/mL M-1-P-producing enzyme was allowed to react with each of the substrates having different chain lengths (glucose, maltose, maltotriose, maltotetraose, maltopentaose, maltohexaose (Seikagaku Corporation)) (2.0%) for one hour at 37°C, and the reaction mixture was subjected to HPLC for analysis in a manner similar to that described in Example 1 (the results are represented as peak area). The retention times of maltooligosaccharides (DP=1 to 11) were estimated through analysis of dextrin (Matsutani Chemical). The results are shown in Table 3.

**Table 3**

| Product (Peak area x 10⁶) | Substrate | | | | | | |
|---|---|---|---|---|---|---|---|
| | Glucose | Maltose | DP=3 | DP=4 | DP=5 | DP=6 | DP=7 |
| Glucose | 65.97 | 0.19 | 0.52 | 0.19 | 0.09 | 0.05 | 0.11 |
| Maltose | 0.00 | 46.79 | 1.84 | 0.15 | 0.27 | 0.61 | 0.28 |
| DP=3 | 0.00 | 0.27 | 33.27 | 1.25 | 0.51 | 0.37 | 0.12 |
| DP=4 | 0.00 | 0.00 | 0.12 | 30.80 | 0.58 | 10.20 | 0.19 |
| DP=5 | 0.00 | 0.00 | 0.00 | 0.00 | 26.40 | 0.00 | 9.59 |
| DP=6 | 0.00 | 0.00 | 0.00 | 0.36 | 0.00 | 8.87 | 0.00 |
| DP=7 | 0.00 | 0.00 | 0.00 | 0.00 | 0.52 | 0.00 | 6.36 |
| DP=8 | 0.00 | 0.00 | 0.00 | 0.00 | 0.00 | 5.04 | 0.00 |
| DP=9 | 0.00 | 0.00 | 0.00 | 0.00 | 0.00 | 0.00 | 3.42 |
| DP=10 | 0.00 | 0.00 | 0.00 | 0.00 | 0.00 | 3.96 | 0.00 |
| DP=11 | 0.00 | 0.00 | 0.00 | 0.00 | 0.00 | 0.00 | 3.47 |

### Example 8 Cloning of M-1-P-producing enzyme gene

*Corynebacterium glutamicum* JCM1321 cells were cultured overnight in a Trypticase Soy Broth medium (BBL) at 30°C. The culture broth (10 mL) was subjected to centrifugation to collect the cells, and the cells were processed through the process described in Manual of Industrial Microbiology and Biotechnolory, 379-391 (1999), ASM press, Washington DC, to thereby prepare chromosomal DNA molecules.

Primer 1 (5'-GAYGGNAAYCCNGCNAARGCNGT (SEQ ID NO: 3)) and primer 2 (5'-GCNGTNGTNGGNGARATHGTNCC (SEQ ID NO: 4)) were designed from Asp-Gly-Asn-Pro-Ala-Lys-Ala-Val and Ala-Val-Val-Gly-Glu-Ile-Val-Pro, respectively, contained in the N-terminal amino acid sequence obtained in Example 3. An LA PCR in vitro cloning kit (product of Takara Bio Inc.) was employed for gene amplification. Specifically, the chromosomal DNA molecules prepared above were cleaved through use of a restriction enzyme HindIII, and the product was ligated to a HindIII cassette attached to the kit. PCR was performed through use of the primer 1 and the cassette primer C1 (30 cycles, 94°C, 30 sec → 60°C, 30 sec → 72°C, 4 min). The reaction mixture was diluted 100-fold, to thereby prepare a template. PCR was performed through use of the template and through use of the primer 2 and a cassette primer C2 (30 cycles, 94°C, 30 sec → 60°C, 30 sec → 72°C, 4 min). The amplified fragments were found to have a length of about 2 kb. The fragments were found to have a nucleotide sequence encoding the N-terminal amino acid sequence obtained in Example 3. Further, in order to obtain an upstream region of the 2-kb fragment, the chromosomal DNA molecules were cleaved through use of EcoRI, and the product was ligated to an EcoRI cassette. The first PCR was performed through use of primer 3 (5'-TGAATAGGCTCAGCCGCCACTGAAGAATCC (SEQ ID NO: 5)) and cassette primer C1 for the upstream region, and for downstream region, primer 4 (5'-TCAGATCATCGCCTACTCCAAGGTTGAT (SEQ ID NO: 6)) and cassette primer C1 were used. The thus-obtained reaction mixture was diluted 100-fold, to thereby prepare a template. The second PCR was performed through use of primer 5 (5'-ACGCCACACAATAGCCGAGACAGG (SEQ ID NO: 7)) for the upstream region and through use of primer 6 (5'-CTGTGGTCAGAGACGAACTTTGTCCGCCTC (SEQ ID NO: 8)) and cassette primer C2 for down stream region (30 cycles, 94°C, 30 sec → 60°C, 30 sec → 72°C, 4 min). The thus-amplified upstream and downstream fragments were found to have a length of about 0.4 kb and about 1 kb, respectively. The sequences of the thus-obtained fragments were determined. Through use of primer 7 (5'-GGAGAGATTCGTCATTGAGTTCACTCG (SEQ ID NO: 9)) designed from the sense chain in the upstream region and primer 8 (5'-TCAGCCCGCTCGCGGTGACCTAAGTC (SEQ ID NO: 10)) designed from the antisense chain, fragments having a length of about 2.6 kb were amplified by use of the chromosomal DNA as a template by means of Pyrobest polymerase (Takara Bio Inc.) (25 cycles, 94°C, 30 sec → 55°C, 30 sec → 72°C, 3 min). The entire nucleotide sequence of the fragments are shown in SEQ ID NO: 1. The sequence was found to have a 2025-bp open reading frame region encoding deducted 675 amino acid residues.

The 675 amino acid residues deducted from the nucleotide sequence was subjected to BLASTP homology search in the NCBI database, revealing that the sequence exhibits 99% to 38% homology with proteins originating from *Corynebacterium glutamicum* ATCC13032; 99%, *Corynebacterium efficiens* YS-314; 84%, *Corynebacterium diphtheriae;* 66%, *Mycobacterium smegmatis;* 60%, *Mycobacterium tuberculosis* CDC1551; 58%, *Mycobacterium bovis* subsp. *Bovis* AF2122/97; 58%, *Mycobacterium avium* subsp. *Paratuber culosisstr.* K10; 58%, *Streptomyces avermitilis* MA-4680; 53%, Thermobifida fusca; 53%, *Streptomyces coelicolor* A3 (2); 52%, *Pseudomonas aeruginosa* PA01; 43%, *Pseudomonas aeruginosa* UCBPP-PA14; 43%, *Pseudomonas syringae pv.* tomato str. DC3000; 42%, *Chloroflexus* aurantiacus; 42%, *Chlorobium tepidum* TLS; 41%, *Bifidobacterium Longum* NCC2705; 39%, *Rhodospirillum* rubrum; 42%, Ralstonia solanacearum; 41%, Bifidobacter *iumLongum* DJ010A; 39%, *Pseudomonas* putida KT2440; 44%, Rhodopseudomonas *palustris;* 41%, Azotobacter *vinelandii*; 43%, *Burkholderia fungorum;* 42%, Rhodobacter *sphaeroides;* 41%, *Pseudomonas* fluorescens Pf0-1; 42%, Xanthomonas campestris str. ATCC33913; 42%, Xanthomonas *axonopodis pv. ctri str.* 306; 41%, *Bordetella bronchiseptica* RB50; 40%, and *Bradyrhizobium japonicum* USDA110; 38%. These proteins are classified into the Family 13 of Glycosyl Hydrolase in the saccharide-related enzyme database CAZy, and deduced to be glycosidase, amylase, glucanase, or the like. However, the deduction is merely based on the genomic sequence of these proteins. Genes of the proteins have never been isolated, and genes to produce the proteins has never been expressed. Thus, nothing has been known in relation to the function. Comparison with the amino acid sequences in terms of alignment reveals highly conserved regions of the 342nd to 352nd amino acid residues (Ara-Glu-Asn-Pro-Pro-Lys-Lys(or Arg)-Tyr-Gln(or Glu)-Asp-Ile) [see the 342nd to 352nd of SEQ ID NO: 2] and the 383rd to 392nd amino acid residues (Phe-Arg-Val(or Ile)-Asp-Asn-Pro-His-Thr-Lys-Pro) [see the 383rd to 392nd of SEQ ID NO: 2] from the N-terminus of the amino acid sequence of the M-1-P-producing enzyme of the present invention.

### Example 9 Expression in E. coli

The 2.6-kb fragment obtained in Example 8 was inserted into an E. *coli* expression vector pUC19 at the SmaI site thereof. Specifically, the PCR fragment (2.5 µg) is treated with T4-polynucleotide kinase (Takara Bio Inc.) to thereby phosphorylate the end, and pUC19 (1 µg) was cleaved through use of SmaI and then treated with alkaline phosphatase (Roche). The both fragments were treated for 30 minutes at 65°C for deactivation of the enzyme, then mixed together, and subjected to ethanol precipitation. After drying under reduced pressure, the fragments were ligated together through use of a ligation kit Ver2 (Takara Bio Inc.). Transformation was performed through use of an *Escherichia* coli JM109 Competent Cells (Takara Bio Inc.), and proliferation was performed in an LB agar medium (1% yeast extract (Difco), 0.5% trypton (Difco), 1% sodium chloride, 1% agar, 25 µg/mL ampicillin). The formed colonies were subjected to shaking culture overnight at 37°C in an LB medium (1% yeast extract (Difco), 0.5% trypton (Difco), 1% sodium chloride, 25 µg/mL ampicillin). The cells were collected. Preparation of plasmid was performed through use of a plasmid isolation kit (Roche). A plasmid (pUMP2) into which the fragment is inserted in the same direction as that of the LacZ promoter of pUC19 and a plasmid (pUMP3) into which the fragment is inserted in the direction opposite to that of the LacZ promoter were obtained.

Each of transformants of the plasmids was cultured overnight at 37°C in an LB medium (2 mL) containing 25 µg/mL ampicillin, the medium being supplemented with or without 1mM IPTG (isopropyl 1-thio-β-D-galactoside). The cultured cells were collected, suspended in 0.5 mL of 50mM phosphate buffer (pH 7), and pulverized by means of an ultrasound pulverizer. The resultant solution was measured in terms of M-1-P producing activity in a manner similar to that described in Example 1. The results are shown in Table 4.

**Table 4**

| Plasmid | Medium | Maltose-1-phosphate producing acitivity (U/L) in culture broth |
|---|---|---|
| pUMP2 | IPTG- | 24.1 |
| pUMP2 | IPTG+ | 41.5 |
| pUMP3 | IPTG- | 11.7 |
| pUMP3 | IPTG+ | 13.6 |

### Example 10 Method for producing M-1-P

The cell strains employed were *Corynebacterium* sp. JCM1300, *Corynebacterium flavescens* JCM1317, *Corynebacterium* glutamicum JCM1318, *Corynebacterium hoagii* JCM1319, *Corynebacterium* glutamicum JCM1321, *Corynebacterium* vitaeruminis JCM1323, Corynebacterium pilosum JCM3714, *Corynebacterium amycolatum* JCM7447, Corynebacterium matruchotii JCM9386, *Corynebacterium* minutissimum JCM9387, *Corynebacterium* striatum JCM9390, and *Corynebacterium* callunae IF015359. Each of the cells was seeded in an SCD agar plate (Nihon Pharmaceutical Co., Ltd.) and incubated overnight at 30°C. Seed culture was performed through the following steps; 10 mL of 0.67% Yeast Nitrogen Base (Difco) was added to a large test tube, one platinum loop of the cell was seeded in the test tube, and shaking culture was performed overnight at 30°C, at 250 rpm.

Primary culture was performed through shaking culture for 6 days at 35°C, at 250 rpm in a medium containing 0.67% Yeast Nitrogen Base (Difco), 10% dextrin MAX 1000 (Matsutani Chemical), and 400mM potassium phosphate buffer (pH 7.0).

M-1-P contained in the supernatant was quantified by means of a DX500 chromatography system (DIONEX).

Quantification was performed through use of the following; column: CarboPac PA1 (Nippon Dionex K.K. 4x250 mm), detector: ED40 pulsed amperometric detector, eluent: solution A; 100mM sodium hydroxide, solution B; 100mM sodium hydroxide containing 1M sodium acetate.

Linear gradient analysis was employed (initial concentration: solution A 90% : solution B 10%, 0-17 min.: solution A 18.5% : solution B 81.5%). As standard substances, 100mM and 50mM M-1-P products (product of SIGMA) were employed. A peak was observed at about 15.5 minutes. The supernatant of the culture broth was diluted 200-fold, and 20 µL of the dilution was injected. The analysis results are shown in Table 5.

**Table 5**

| Strain | Amount of M-1-P produced (g/L) |
|---|---|
| *Corynebacterium* sp. JCM1300 | 1.5 |
| *Corynebacterium flavescens* JCM1317 | 0.2 |
| *Corynebacterium glutamicum* JCM1318 | 0.7 |
| *Corynebacterium hoagii* JCM1319 | 1.8 |
| *Corynebacterium glutamicum* JCM1321 | 3.1 |
| *Corynebacterium vitaeruminis* JCM1323 | 0.3 |
| *Corynebacterium* pilosum JCM3714 | 0.2 |
| Corynebacterium *amycolatum* JCM7447 | 0.1 |
| *Corynebacterium matruchotii* JCM9386 | 0.1 |
| *Corynebacterium minutissimum JCM9387* | 0.2 |
| *Corynebacterium* striatum JCM9390 | 0.3 |
| *Corynebacterium callunae* IFO15359 | 4.0 |

The results indicate that M-1-P can be produced by culturing a bacterium belonging to the family *Corynebacterium* in a medium containing phosphate and an oligosaccharide or polysaccharide having an α-1,4-glycosidic bond. The supernatant of the culture broth may also contain, in addition to M-1-P, glucose-1-phosphate or a similar phosphorylated saccharide produced. Therefore, M-1-P was isolated by separating and removing the cells, subjecting the supernatant to ultrafiltration, reverse osmosis membrane, electrodialysis, ion exchanged membrane, ion exchanged resin, active carbon, synthetic absorbent, crystallization, or a similar treatment in accordance with needs, and then subjecting to ion exchange chromatography.

### Referential Example 1 Production of phophorylase through use of a bacterium belonging to the family Corynebacterium

Each of *Corynebacterium vitaeruminis* JCM1323 and *Corynebacterium callunae* IFO15359 cells was seeded in an SCD agar medium (Nihon Pharmaceutical Co., Ltd.) and cultured at 30°C. One platinum loop of the thus-obtained bacterium was seeded in a liquid medium (0.67% Yeast Nitrogen Base (Difco ), each of 50, 100, and 200mM phosphate buffers (pH 7), 15% dextrin (sigma, originating from potato)), and subjected to shaking culture for 6 days at 30°C. The cells contained in the culture broth were removed through centrifugation, and the maltodextrin phosphorylase activity of the supernatant was measured.

The maltodextrin phosphorylase activity of the supernatant was measured through a partially-modified method of Weinhausel (Enzyme Microb. Technol., 17, 140-146 (1995)). Specifically, to a 96-well microplate, were added each of the culture broth supernatant samples (20 µL) which had been appropriately diluted and an enzyme reaction solution. (180 µL) (200mM potassium phosphate buffer (pH 7.0), 2% dextrin, 100mM Tris-acetate buffer (pH 6.8), 2mM EDTA, 10mM magnesium sulfate, 2mM NAD, 10µM glucose-1,6-diphosphate, 1.2 unit/mL phosphoglucomutase (originating from rabbit muscle, product of Roche Diagnostics K.K.), 1.2 unit/mL glucose-6-phosphate dehydrogenase (originating from Leuconostoc mesenteroides, product of Roche Diagnostics K.K.)). Increase in absorbance at 340 nm attributed to G1P production was measured at 37°C. One enzyme unit (U) is defined as the amount of enzyme that produces 1 µmol of glucose-1-phosphate (G1P) per minute. The results are shown in Table 6.

**Table 6**

| Extracellular maltodextrin phosphorylase (U/L) | | | |
|---|---|---|---|
| Phosphate concentration | 50 | 100 | 200 |
| C. *vitaeruminis* | 7.1 | 134.0 | 90.3 |
| C. *callunae* | 0 | 3.6 | 42.0 |

As shown in Table 6, by increasing phosphate concentration in medium, maltodextrin phosphorylase was extracellularlly released. Thus, when the region of the gene encoding the enzyme was removed from the genome, or the enzyme is deactivated, only M-1-P can be produced without production of glucose-1-phosphate.

## Claims

1. A method for producing maltose-1-phosphate, **characterized by** causing a maltose-1-phosphate-producing enzyme, having a homology of 90% or more to an amino acid sequence according to SEQ ID No. 2, which is capable of producing maltose-1-phosphate from a phosphoric acid or a salt thereof and an oligosaccharide or polysaccharide containing an α-1,4-glycosidic bond and having a glucose polymerization degree of 5 or more, to act on a phosphoric acid or a salt thereof and an oligosaccharide or polysaccharide having a glucose polymerization degree of 5 or more and containing an α-1,4-glycosidic bond.

2. The method of claim 1, wherein the maltose-1-phosphate-producing enzyme has the following enzymatic characteristics;
1) Substrate specificity: in the presence of a phosphoric acid or a salt thereof, effectively acting on oligosaccharide or polysaccharide containing an α-1,4-glucosidic bond and having a glucose polymerization degree of 6 or more, or a degraded product thereof, thereby producing maltose-1-phosphate; weakly acting on an oligosaccharide having a glucose polymerization degree of 5; and exhibiting substantially no action on an oligosaccharide having a polymerization degree of 2 to 4,
2) Molecular weight: about 75 kDa (SDS-PAGE),
3) Optimum pH: 6.5 to 8.0, and
4) Optimum temperature: 35 to 50°C.

3. The method of claim 1 or 2 wherein the maltose-1-phosphate-producing enzyme has an amino acid sequence represented by SEQ ID NO: 2.

4. The method of any one of claims 1 to 3 wherein the maltose-1-phosphate-producing enzyme is encoded by a gene having the following DNA (a) or (b);
(a) a DNA which has a nucleotide sequence represented by SEQ ID NO: 1 which encodes a protein capable of producing maltose-1-phosphate from a phosphoric acid or a salt thereof and an oligosaccharide or polysaccharide containing an α-1,4-glycosidic bond and having a glucose polymerization degree of 5 or more;
(b) a DNA which is capable of hybridizing a DNA fragment having the nucleotide sequence of (a) under stringent conditions and which encodes a protein capable of producing maltose-1-phosphate from a phosphoric acid or a salt thereof and an oligosaccharide or polysaccharide containing an α-1,4-glycosidic bond and having a glucose polymerization degree of 5 or more.

5. The method for producing maltose-1-phosphate according to any one of claims 1 to 3, comprising culturing a microorganism capable of producing the maltose-1-phosphate-producing enzyme in a medium containing 1 mM or more of a phosphoric acid or a salt thereof and an oligosaccharide or polysaccharide having a glucose polymerization degree of 5 or more and containing an α-1,4-glycosidic bond, and collecting maltose-1-phosphate produced and accumulated in the medium.

6. The method according to claim 5, wherein the microorganism contains a recombinant vector containing a maltose-1-phosphate-producing enzyme gene having the following DNA (a) or (b);
(a) a DNA which has a nucleotide sequence represented by SEQ ID NO: 1 which encodes a protein capable of producing maltose-1-phosphate from a phosphoric acid or a salt thereof and an oligosaccharide or polysaccharide containing an α-1,4-glycosidic bond and having a glucose polymerization degree of 5 or more;
(b) a DNA which is capable of hybridizing a DNA fragment having the nucleotide sequence of (a) under stringent conditions and which encodes a protein capable of producing maltose-1-phosphate from a phosphoric acid or a salt thereof and an oligosaccharide or polysaccharide containing an α-1,4-glycosidic bond and having a glucose polymerization degree of 5 or more.

7. The method according to claim 5, wherein the microorganism is a bacterium belonging to the family *Corynebacterium.*

8. The method according to any one of claims 5 to 7,
wherein the concentration of a phosphoric acid or a salt thereof is 50 mM to 2,000 mM.

9. A maltose-1-phosphate-producing enzyme consisting of the amino acid sequence according to SEQ ID No. 2, which is capable of producing maltose-1-phosphate from a phosphoric acid or a salt thereof and an oligosaccharide or polysaccharide containing an α-1,4-glycosidic bond and having a glucose polymerization degree of 5 or more.

10. A gene encoding the protein according to claim 9.

11. The gene according to claim 10 represented by SEQ ID NO: 1, which encodes a protein capable of producing maltose-1-phosphate from a phosphoric acid or a salt thereof and an oligosaccharide or polysaccharide containing an α-1,4-glycosidic bond and having a glucose polymerization degree of 5 or more.

12. A recombinant vector containing a gene according to claim 10 or 11.

13. A transformant containing a recombinant vector according to claim 12.

14. The transformant according to claim 13, wherein the host is a microorganism.

## Patentansprüche

1. Verfahren zum Herstellen von Maltose-1-Phosphat, **dadurch gekennzeichnet, dass** verursacht wird, dass ein Maltose-1-Phosphat-herstellendes Enzym mit einer Homologie von 90 % oder mehr zu einer Aminosäuresequenz gemäß SEQ ID NO: 2, welches befähigt ist, Maltose-1-Phosphat aus Phosphorsäure oder einem Salz davon und einem Oligosaccharid oder Polysaccharid, das eine α-1,4-glycosidische Bindung enthält und einen Glukosepolymerisationsgrad von 5 oder mehr aufweist, herzustellen, auf eine Phosphorsäure oder ein Salz davon und ein Oligosaccharid oder Polysaccharid mit einem Glukosepolymerisationsgrad von 5 oder mehr, das eine α-1,4-glycosidische Bindung enthält, einwirkt.

2. Verfahren gemäß Anspruch 1, wobei das Maltose-1-Phosphat-herstellende Enzym die folgenden enzymatischen Eigenschaften hat:
1) Substratspezifität: in Gegenwart einer Phosphorsäure oder eines Salzes davon wirksames Einwirken auf ein Oligosaccharid oder Polysaccharid, das eine α-1,4-glucosidische Bindung enthält und das einen Glukosepolymerisationsgrad von 6 oder mehr aufweist oder auf ein davon abgebautes Produkt, wobei Maltose-1-Phosphat hergestellt wird; schwaches Einwirken auf ein Oligosaccharid, das einen Glukosepolymerisationsgrad von 5 aufweist; und im Wesentlichen keine Einwirkung auf ein Oligosaccharid, das einen Polymerisationsgrad von 2 bis 4 aufweist,
2) Molekulargewicht: ungefähr 75 kDa (SDS-PAGE),
3) pH-Optimum: 6,5 bis 8,0, sowie
4) Temperaturoptimum: 35 bis 50°C.

3. Verfahren gemäß Anspruch 1 oder 2, wobei das Maltose-1-Phosphat-herstellende Enzym eine Aminosäuresequenz hat, die durch SEQ ID NO: 2 dargestellt wird.

4. Verfahren gemäß einem der Ansprüche 1 bis 3, wobei das Maltose-1-Phosphat-herstellende Enzym durch ein Gen mit der folgenden DNA (a) oder (b) kodiert wird;
(a) einer DNA, die eine Nukleotidsequenz hat, die durch SEQ ID NO: 1 dargestellt wird, die ein Protein kodiert, das befähigt ist, Maltose-1-Phosphat aus einer Phosphorsäure oder einem Salz davon und einem Oligosacccharid oder Polysaccharid, das eine α-1,4-glycosidische Bindung enthält und einen Glukosepolymerisationsgrad von 5 oder mehr aufweist, herzustellen;
(b) einer DNA, die befähigt ist, mit einem DNA-Fragment mit der Nukleotidsequenz gemäß (a) unter stringenten Bedingungen zu hybridisieren und die ein Protein kodiert, das befähigt ist, Maltose-1-Phosphat aus einer Phosphorsäure oder einem Salz davon und einem Oligosaccharid oder Polysaccharid, das eine α-1,4-glycosidische Bindung enthält und einen Glukosepolymerisationsgrad von 5 oder mehr aufweist, herzustellen.

5. Verfahren zum Herstellen von Maltose-1-Phosphat gemäß einem der Ansprüche 1 bis 3, umfassend das Kultivieren eines Mikroorganismus, der befähigt Lage ist, das Maltose-1-Phosphat-herstellende Enzym herzustellen, in einem Medium, enthaltend 1 mM oder mehr einer Phosphorsäure oder eines Salzes davon und ein Oligosaccharid oder Polysaccharid, das einen Glukosepolymerisationsgrad von 5 oder mehr aufweist und eine α-1,4-glycosidische Bindung enthält, sowie Gewinnen von Maltose-1-Phosphat, das hergestellt und im Medium angesammelt wurde.

6. Verfahren gemäß Anspruch 5, wobei der Mikroorganismus einen rekombinanten Vektor enthält, der ein Gen für ein Maltose-1-Phosphat-herstellendes Enzym enthält, und der die folgenden DNA (a) oder (b) aufweist;
(a) eine DNA, die eine Nukleotidsequenz hat, die durch SEQ ID NO: 1 dargestellt wird, die ein Protein kodiert, das befähigt ist, Maltose-1-Phosphat aus einer Phosphorsäure oder einem Salz davon und einem Oligosacccharid oder Polysaccharid, das eine α-1,4-glycosidische Bindung enthält und einen Glukosepolymerisationsgrad von 5 oder mehr aufweist, herzustellen;
(b) eine DNA, die befähigt ist, mit einem DNA-Fragment mit der Nukleotidsequenz von (a) unter stringenten Bedingungen zu hybridisieren und die ein Protein kodiert, das befähigt ist, Maltose-1-Phosphat aus einer Phosphorsäure oder einem Salz davon und einem Oligosaccharid oder Polysaccharid, das eine α-1,4-glycosidische Bindung enthält und einen Glukosepolymerisationsgrad von 5 oder mehr aufweist, herzustellen.

7. Verfahren gemäß Anspruch 5, wobei der Mikroorganismus ein Bakterium ist, das zur Familie Corynebacterium gehört.

8. Verfahren gemäß einem der Ansprüche 5 bis 7, wobei die Konzentration einer Phosphorsäure oder eines Salzes davon 50 mM bis 2.000 mM beträgt.

9. Maltose-1-Phosphat-herstellendes Enzym, bestehend aus der Aminosäuresequenz gemäß SEQ ID NO: 2, welches befähigt ist, Maltose-1-Phosphat aus einer Phosphorsäure oder einem Salz davon und einem Oligosaccharid oder Polysaccharid, das eine α-1,4-glycosidische Bindung enthält und einen Glukosepolymerisationsgrad von 5 oder mehr aufweist, herzustellen.

10. Gen, das das Protein gemäß Anspruch 9 kodiert.

11. Gen gemäß Anspruch 10, dargestellt durch SEQ ID NO: 1, welches ein Protein kodiert, das befähigt ist, Maltose-1-Phosphat aus einer Phosphorsäure oder einem Salz davon und einem Oligosaccharid oder Polysaccharid, das eine α-1,4-glycosidische Bindung enthält und einen Glukosepolymerisationsgrad von 5 oder mehr aufweist, herzustellen.

12. Rekombinanter Vektor, der ein Gen gemäß Anspruch 10 oder 11 enthält.

13. Transformante, die einen rekombinanten Vektor gemäß Anspruch 12 enthält.

14. Transformante gemäß Anspruch 13, wobei der Wirt ein Mikroorganismus ist.

## Revendications

1. Procédé pour produire du maltose-1-phosphate, caractérisé en amenant une enzyme de production de maltose-1-phosphate, ayant une homologie de 90 % ou plus avec une séquence d'acides aminés selon SEQ ID n° 2, qui est susceptible de produire du maltose-1-phosphate à partir d'un acide phosphorique ou un sel de ce dernier et d'un oligosaccharide ou polysaccharide contenant une liaison α-1,4-glycosidique et ayant un degré de polymérisation de glucose de 5 ou plus, à agir sur un acide phosphorique ou un sel de ce dernier et sur un oligosaccharide ou polysaccharide ayant un degré de polymérisation de glucose de 5 ou plus et contenant une liaison α-1,4-glycosidique.

2. Procédé selon la revendication 1, dans lequel l'enzyme de production de maltose-1-phosphate a les caractéristiques enzymatiques suivantes ;
1) Spécificité de substrat : en présence d'un acide phosphorique ou un sel de ce dernier, action efficace sur un oligosaccharide ou polysaccharide contenant une liaison α-1,4-glucosidique et ayant un degré de polymérisation de glucose de 6 ou plus, ou un produit dégradé de ce dernier, produisant de ce fait du maltose-1-phosphate ; action faible sur un oligosaccharide ayant un degré de polymérisation de glucose de 5 ; et sensiblement aucune action sur un oligosaccharide ayant un degré de polymérisation de 2 à 4,
2) Masse moléculaire : environ 75 kDa (SDS-PAGE),
3) pH optimal : 6,5 à 8,0, et
4) Température optimale : 35 à 50 °C.

3. Procédé selon la revendication 1 ou 2, dans lequel l'enzyme de production de maltose-1-phosphate a une séquence d'acides aminés représentée par SEQ ID n° 2.

4. Procédé selon l'une quelconque des revendications 1 à 3, dans lequel l'enzyme de production de maltose-1-phosphate est codée par un gène ayant l'ADN suivant (a) ou (b) ;
(a) un ADN qui a une séquence de nucléotides représentée par SEQ ID n° 1 qui code une protéine susceptible de produire du maltose-1-phosphate à partir d'un acide phosphorique ou un sel de ce dernier et d'un oligosaccharide ou polysaccharide contenant une liaison α-1,4-glycosidique et ayant un degré de polymérisation de glucose de 5 ou plus ;
(b) un ADN qui est susceptible d'hybrider un fragment d'ADN ayant la séquence de nucléotides de (a) sous des conditions rigoureuses et qui code une protéine susceptible de produire du maltose-1-phosphate à partir d'un acide phosphorique ou un sel de ce dernier et d'un oligosaccharide ou polysaccharide contenant une liaison α-1,4-glycosidique et ayant d'un degré de polymérisation de glucose de 5 ou plus.

5. Procédé pour produire du maltose-1-phosphate selon l'une quelconque des revendications 1 à 3, comprenant la culture d'un micro-organisme susceptible de produire l'enzyme de production de maltose-1-phosphate dans un milieu contenant 1 mM ou plus d'un acide phosphorique ou un sel de ce dernier et d'un oligosaccharide ou polysaccharide ayant un degré de polymérisation de glucose de 5 ou plus et contenant une liaison α-1,4-glycosidique, et la collecte du maltose-1-phosphate produit et accumulé dans le milieu.

6. Procédé selon la revendication 5, dans lequel le micro-organisme contient un vecteur recombinant contenant un gène d'enzyme de production de maltose-1-phosphate ayant l'ADN suivant (a) ou (b) ;
(a) un ADN qui a une séquence de nucléotides représentée par SEQ ID n° 1 qui code une protéine susceptible de produire du maltose-1-phosphate à partir d'un acide phosphorique ou un sel de ce dernier et d'un oligosaccharide ou polysaccharide contenant une liaison α-1,4-glycosidique et ayant un degré de polymérisation de glucose de 5 ou plus ;
(b) un ADN qui est susceptible d'hybrider un fragment d'ADN ayant la séquence de nucléotides de (a) sous des conditions rigoureuses et qui code une protéine susceptible de produire du maltose-1-phosphate à partir d'un acide phosphorique ou un sel de ce dernier et d'un oligosaccharide ou polysaccharide contenant une liaison α-1,4-glycosidique et ayant un degré de polymérisation de glucose de 5 ou plus.

7. Procédé selon la revendication 5, dans lequel le micro-organisme est une bactérie appartenant à la famille *Corynebacterium.*

8. Procédé selon l'une quelconque des revendications 5 à 7, dans lequel la concentration d'un acide phosphorique ou un sel de ce dernier est de 50 mM à 2 000 mM.

9. Enzyme de production de maltose-1-phosphate constituée par la séquence d'acides aminés selon SEQ ID n° 2, qui est susceptible de produire du maltose-1-phosphate à partir d'un acide phosphorique ou un sel de ce dernier et d'un oligosaccharide ou polysaccharide contenant une liaison α-1,4-glycosidique et ayant un degré de polymérisation de glucose de 5 ou plus.

10. Gène codant la protéine selon la revendication 9.

11. Gène selon la revendication 10, représenté par SEQ ID n° 1, qui code une protéine susceptible de produire du maltose-1-phosphate à partir d'un acide phosphorique ou un sel de ce dernier et d'un oligosaccharide ou polysaccharide contenant une liaison α-1,4-glycosidique et ayant un degré de polymérisation de glucose de 5 ou plus.

12. Vecteur recombinant contenant un gène selon la revendication 10 ou 11.

13. Transformé contenant un vecteur recombinant selon la revendication 12.

14. Transformé selon la revendication 13, dans lequel l'hôte est un micro-organisme.
